Europäisches Patentamt

(19) European Patent Office

Office européen des brevets

(11) Veröffentlichungsnummer: **0 056 094**
A1

# (12) EUROPÄISCHE PATENTANMELDUNG

(21) Anmeldenummer: 81109038.0

(22) Anmeldetag: 27.10.81

(51) Int. Cl.³: **C 01 B 33/32,** C 01 B 33/12, B 01 J 21/08, B 01 J 23/02, B 01 J 20/10, C 08 K 3/36

(30) Priorität: 14.01.81 DE 3100942

(43) Veröffentlichungstag der Anmeldung: 21.07.82 Patentblatt 82/29

(84) Benannte Vertragsstaaten: **AT BE CH DE FR GB IT LI LU NL SE**

(71) Anmelder: **Matouschek, Reinhard, Dr., Semerstrasse 10, D-8920 Schongau (DE)**

(72) Erfinder: **Matouschek, Reinhard, Dr., Semerstrasse 10, D-8920 Schongau (DE)**

(74) Vertreter: **Kraus, Walter, Dr. et al, Patentanwälte Dres. Kraus & Weisert Irmgardstrasse 15, D-8000 München 71 (DE)**

(54) Verfahren zur Herstellung kristalliner Natriumsilicate und kristallisierter Formen des hydratisierten Siliciumdioxids, die bei dem Verfahren erhaltenen Produkte sowie ihre Verwendung.

(57) Die Erfindung betrifft ein Verfahren zur Herstellung kristalliner Natriumsilicate und kristallisierter Formen des hydratisierten Siliciumdioxids durch Erhitzen von Natriumcarbonat und Quarz bei erhöhter Temperatur.

Gemäß dem erfindungsgemäßen Verfahren werden Natriumcarbonat der Körnung bis 2 mm und Quarzpulver mit einer Oberfläche nach BET von 0,03 bis 20 m²/g im Molverhältnis 1 : 1,9 bis 1 : 3 intensiv miteinander vermischt und dann bei Temperaturen zwischen 400 und 900 °C drei bis zehn Stunden getempert oder bei Temperaturen zwischen 900 und 1100 °C eine halbe bis drei Stunden zu Glas geschmolzen, wobei das Glas dann abgekühlt und das erstarrte Glas erneut vier bis neun Stunden bei Temperaturen zwischen 400 und 860 °C getempert wird.

Danach wird das erhaltene Natriumsilicat isoliert und zur Herstellung kristallisierter Formen des hydratisierten Siliciumdioxids mit Säure behandelt.

Die Erfindung betrifft weiterhin die bei dem Verfahren erhaltenen Produkte sowie die Verwendung dieser Produkte als Füllstoffe, Trägerstoffe, Katalysatoren und Katalysatorträger.

EP 0 056 094 A1

BESCHREIBUNG

Die Erfindung betrifft ein neues Verfahren zur Herstellung kristalliner Natriumsilicate und kristallisierter Formen des hydratisierten Siliciumdioxids, die bei diesem Verfahren erhaltenen Produkte sowie ihre Verwendung.

Neben einigen kristallinen und amorphen Natriumsilicaten und kristallisierten Formen von hydratisierten Siliciumdioxiden kennt man zahlreiche handelsübliche Formen von amorphem Siliciumdioxid, deren Hydratisierungsgrad (Wassergehalt), spezifische Oberfläche, Porenweite und Teilchengröße innerhalb sehr weiter Grenzen schwanken. Ihre industrielle Anwendung wird durch die spezifische Oberfläche, ihre Teilchengröße und ihren Wassergehalt bestimmt. Eine häufig im Handel angebotene Form von hydratisiertem Siliciumdioxid ist die gefällte und die pyrogene Kieselsäure, die amorph ist, mit diversen spezifischen Oberflächen und Teilchengrößen industriell hergestellt und in sehr unterschiedlichen Bereichen eingesetzt wird, zum Beispiel als verstärkender Füllstoff in Natur- und Synthesekautschuk, als Trägerstoff für Öle pflanzlicher und tierischer Herkunft und für Biozide, wie auch für selektive Adsorption und Verdickung.

Gefällte Kieselsäuren werden durch Umsetzen einer wäßrigen Lösung von Alkalisilicat, insbesondere Natriumsilicat, mit einer Mineralsäure (wie zum Beispiel Schwefelsäure, Salzsäure oder Kohlensäure) gewonnen. Dabei werden die Reaktionspartner durch kräftiges Rühren miteinander vermischt. Die Kieselsäure fällt als weißer Niederschlag aus, der auf einem Filteraggregat eingedickt und mit Wasser salzarm bis salzfrei gewaschen wird. Nach anschließender Trocknung des Filterrückstands wird die gewünschte Teilchengrößenverteilung durch geeignete Vermahlung vorgewonnen.

Wasserglaslösung wird hergestellt, indem Natriumcarbonat oder Natriumhydroxid mit grobem Quarzpulver vermischt und anschließend einem langdauernden Schmelzprozeß unterzogen werden. Das gewonnene Natriumsilicatglas wird anschließend in Wasser aufgelöst.

Pyrogene Kieselsäuren werden durch Hydrolyse von Halogensilanen in einer Wassserstofflamme hergestellt. Die Halogensilane werden durch Einwirken der Halogene oder Halogenwasserstoffe auf Silicium oder eine Mischung aus Siliciumdioxid und Kohlenstoff gewonnen.

Aus kristallinen Alkalisilicaten mit Schichtstruktur lassen sich die entsprechenden kristallisierten Formen des hydratisierten Siliciumdioxids (genaue Bezeichnung: "kristalline Kieselsäure") durch Austausch der Alkaliionen gegen Protonen mit Hilfe von Mineralsäuren erhalten (vgl. F. Wodtcke und F. Liebau "Zeitschrift für anorg. und allg. Chemie", Band 335, Seite 178, 1965). Röntgenographische Untersuchungen haben bewiesen, daß die Schichtstruktur des Alkalisilicats erhalten bleibt. Durch Einsatz unterschiedlicher Ausgangssilicate werden dementsprechend auch unterschiedliche kristallisierte Formen des hydratisierten Siliciumdioxids gewonnen. Zwischen die Schichten der Kieselsäure können Gastmoleküle spezifisch aufgenommen werden, eine Eigenschaft, die bei gefällten und pyrogenen Kieselsäuren nicht vorhanden ist. Darüberhinaus können bei kristallisierten Formen des hydratisierten Siliciumdioxids Silanolgruppendichten bis zu 6,6 Silanolgruppen pro nm² erreicht werden gegenüber maximal 5,0 Silanolgruppen pro nm² bei gefällten Kieselsäuren.

Als Ausgangsverbindungen für die Synthese der kristallinen Natriumsilicate dienten bisher entweder eine amorphe Form des hydratisierten Siliciumdioxids oder ein entsprechendes Natriumsilicatglas. Die benötigte amorphe Form des hydratisierten Siliciumdioxids wurde durch Umsetzung einer wäßrigen Natriumsilicatlösung mit einer Mineralsäure gewonnen und ist deshalb als Ausgangsmaterial zu teuer. Ebenso wird das benötigte Natriumsilicatglas in einem langandauernden, energieaufwendigen Schmelzprozeß aus einer amorphen Form des hydratisierten Siliciumdioxids oder aus einem grobkörnigen Quarzpulver gewonnen.

Die amorphe Form des hydratisierten Siliciumdioxids wird mit einer wäßrigen Natriumhydroxidlösung mehrere Tage bis Wochen zu kristallinen Natriumsilicaten umgesetzt. Aus amorphen Natriumsilicatgläsern bilden sich kristalline Natriumsilicate nach mindestens eintägigem bis mehrtägigem Tempern bei hohen Temperaturen. Beide Verfahrensschritte verbrauchen aufgrund der benötigten Einsatzstoffe, Temperaturen und der langen Zeitdauer viel Energie.

In der US-PS 4 073 865 und der ihr entsprechenden DE-AS 27 51 443 wird eine kristallisierte Form des hydratisierten Siliciumdioxids beschrieben, das in Anwesenheit von Fluoridionen und eines quaternären Kations erhalten wird. Die Herstellung dieses Produkts ist jedoch technisch aufwendig und teuer.

Der vorliegenden Erfindung liegt die Aufgabe zugrunde, ein neues Herstellungsverfahren für kristalline Natriumsilicate und kristallisierte Formen des hydratisierten Siliciumdioxids zur Verfügung zu stellen. Erfindungsgemäß sollen die Voraussetzungen geschaffen werden, daß diese Produkte wirtschaftlich hergestellt werden können. Für kristallisierte Natriumsilicate und kristallisierte Formen des hydratisierten Siliciumdioxids besteht ein großer Bedarf, da diese Verbindungen im Gegensatz zu den amorphen Formen bei den meisten Anwendungsgebieten der amorphen Formen bessere Ergebnisse zeigen und sie außerdem eine höhere Schüttdichte aufweisen als die amorphen Verbindungen und somit ihre Transport-, Lagerungs- und Handhabungskosten wesentlich geringer sind als bei den entsprechenden amorphen Formen.

Überraschenderweise wurde gefunden, daß sich neue kristallisierte Formen des hydratisierten Siliciumdioxids und ihrer kristallinen Natriumsilicate im Gegensatz zu den bisherigen bekannten Verfahren mit billigeren Einsatzstoffen, in kürzerer Zeit und bei günstigeren Temperaturen, folglich wirtschaftlich, herstellen lassen.

Die Erfindung betrifft somit ein Verfahren zur Herstellung kristalliner Natriumsilicate und kristallisierter Formen des hydratisierten Siliciumdioxids durch Erhitzen von Natriumcarbonat und Quarz bei erhöhter Temperatur, das dadurch gekennzeichnet ist, daß Natriumcarbonat der Körnung bis 2 mm und Quarzpulver mit einer Oberfläche nach BET von 0,03 $m^2$/g bis 20 $m^2$/g im Molverhältnis 1:1,9 bis 1:3 intensiv miteinander vermischt werden, diese Reaktionsmischung

a) bei Temperaturen zwischen 400 und 900°C drei bis zehn Stunden getempert oder

b) bei Temperaturen zwischen 900 und 1100°C eine halbe bis drei Stunden zu Glas geschmolzen wird, das flüssige Glas innerhalb kurzer Zeit abgekühlt und das erstarrte Glas darauf vier bis neun Stunden bei Temperaturen zwischen 400 und 860°C getempert wird

und das nach dem Tempern und Erkalten erhaltene Natriumsilicat isoliert und vermahlen oder zur Herstellung kristallisierter Formen des hydratisierten Siliciumdioxids mit verdünnter Mineralsäure, organischer Säure oder einem Säuregemisch umgesetzt, der entstehende Feststoffbrei abfiltriert, salzarm gewaschen, der Filterkuchen getrocknet und anschließend vermahlen wird.

Die Erfindung betrifft weiterhin die bei dem Verfahren erhaltenen kristallinen Natriumsilicate und kristallisierte Formen des hydratisierten Siliciumdioxids sowie die Verwendung dieser Produkte als verstärkende Füllstoffe in Natur- und Synthesekautschuk, Siliconkautschuk, Kunststoffen, Papier, für kosmetische und medizinische Anwendungen, als Trägerstoffe für Öle pflanzlicher und tierischer Herkunft und Biocide sowie für die selektive Adsorption und Verdickung als Katalysator und Katalysatorträger. Beispiele für die Verwendung auf medizinischem und kosmetischem Gebiet sind kosmetische und medizinische Cremes und Salben, Zahnpasten, Puder und Gesichtsmasken.

Erfindungsgemäß können somit kristallisierte Formen des hydratisierten Siliciumdioxids, zum Beispiel Verbindungen der chemischen Formeln $H_2Si_2O_5$, $H_6Si_8O_{19}$ und $H_2Si_3O_7$, sowie ihre entsprechenden kristallinen Natriumsilicate, zum Beispiel Verbindungen der Formeln $Na_2Si_2O_5$, $Na_6Si_8O_{19}$ und $Na_2Si_3O_7$, wirtschaftlich hergestellt werden. Als Ausgangsverbindungen für die Synthese der kristallinen Natriumsilicate dienen Natriumcarbonat gekörnt (Körnung bis 2 mm) und ein Quarzpulver mit Oberflächen nach BET von $0,03$ $m^2/g$ bis $20$ $m^2/g$, vorzugsweise $0,2$ bis $3,0$ $m^2/g$. Das Quarzpulver fällt beim Abbau von Quarz- und Aluminiumsilicatvorkommen in reichlicher Menge an und muß nur teilweise auf die gewünschte Feinheit aufgemahlen werden. Das Quarzpulver kann im Gegensatz zum Herstellungsverfahren von Natriumsilicatlösung, die zur Gewinnung gefällter Kieselsäuren verwendet wird, bis zu 10% Verunreinigungen in Form von $Al_2O_3$, $Fe_2O_3$, $TiO_2$ u.a. enthalten. Dies ist für das erfindungsgemäße Verfahren von besonderem Vorteil, da es nicht erforderlich ist, wie bei allen bekannten Verfahren reines Quarzpulver einzusetzen.

Das Natriumcarbonat kann bis zu 20 Gew.-% Calciumoxid und/oder Calciumcarbonat und geringe Mengen, d.h. bis zu maximal 5 Gew.-%, Eisen(II,III)-oxid enthalten. Dies ist ein

0056094

weiterer Vorteil des erfindungsgemäßen Verfahrens, da ebenfalls nicht von reinem Natriumcarbonat ausgegangen werden muß. Das Natriumcarbonat kann weiterhin bis zu 90 Mol-%, vorzugsweise 20 bis 50 Mol-%, durch Natriumhydroxid ersetzt werden. Das Natriumhydroxid wird in Form einer wäßrigen Lösung, vorzugsweise einer 30- 50%-igen wäßrigen Lösung, den übrigen Reaktionspartner beigemischt. Die erhaltene Mischung wird anschließend zur Entfernung des Wassers bei erhöhten Temperaturen getrocknet. Die Temperaturen sind nicht kritisch und können vom Fachmann ausgewählt werden und können im Bereich bis zu 200°C oder auch noch darüber liegen.

Den beiden Ausgangsstoffen, Quarzpulver und Natriumcarbonat, können Mineralisatoren, wie beispielsweise Alkali-, Erdalkalihalogenide oder deren Gemische zugesetzt werden. Diese Mineralisatoren werden in Mengen von 0 bis 30 Gew.-%, vorzugsweise 3 bis 10 Gew.-%, zugesetzt. Bevorzugt verwendet man Natriumchlorid. Dadurch kann die Temperatur für die Herstellung der kristallinen Natriumsilicate herabgesetzt werden.

Darüberhinaus lassen sich Entmischungen der Reaktionspartner und daraus resultierende unvollständige Umsetzungen besser verhindern. Auch bei der anschließenden Reaktion der kristallinen Natriumsilicate mit Mineralsäuren, organischen Säuren und Gemischen verschiedener Säuren zu kristallinen Formen des hydratisierten Siliciumdioxids bewirkt die Verwendung von Mineralisatoren bei der Silicatsynthese einen leichteren Zerfall der dicht miteinander verwachsenen Kristallaggregate des Silicats und damit eine höhere Umsetzungsgeschwindigkeit. Die Konzentration der verwendeten Mineralsäure, zum Beispiel Schwefelsäure oder Salzsäure, organischen Säure, beispielsweise Essigsäure, oder ihrer Gemische kann zwischen 0,05 und 10 mol/l, vorzugsweise 1 und 5 mol/l, liegen, so daß auch Abfallsäuren und Säuregemische, wie sie bei vielen industriellen Prozessen entstehen, eingesetzt werden können.

Der Filterkuchen, der bei der Filtration der Kieselsäuresuspension, die bei der Umsetzung des kristallinen Natriumsilicats mit Mineralsäure, organischer Säure und Säuregemisch entsteht, gewonnen wird, enthält je nach den Filtrationsbedingungen 50 bis 70 Gew.% Feststoffgehalt. Dadurch wird wesentlich weniger Trocknungsenergie als bei gefällten Kieselsäuren verbraucht.

Im neuen Verfahren werden Natriumcarbonat und Quarzpulver im Molverhältnis 1:1,9 bis 1:3, vorzugsweise 1:2 oder 1:2,67, miteinander umgesetzt. Bei einem Molverhältnis von 1:2 erhält man praktisch reines $Na_2Si_2O_5$ bzw. reine $H_2Si_2O_5$. Bei einem Molverhältnis von 1:2,67 erhält man praktisch reines $Na_6Si_8O_{19}$ bzw. reine $H_6Si_8O_{19}$. Der Fachmann kann die Molverhältnisse leicht auswählen, je nach den gewünschten Produkten. Zusätzlich können 0 bis 30 Gew.-%, vorzugsweise 3 bis 10 Gew.-%, Mineralisator (bezogen auf die Menge an Natriumcarbonat und Quarz), wie zum Beispiel Natriumchlorid, verwendet werden. Die Reaktionsmasse wird innigst miteinander vermischt. Die Umsetzung dieser Mischung bei Temperaturen zwischen 400 und 900°C, vorzugsweise 700 bis 860°C, ist nach drei bis zehn Stunden, vorzugsweise vier bis sechs Stunden, beendet. Man erhält kristallisierte Natriumcilicate.

Zur Herstellung der kristallisierten Formen des hydratisierten Siliciumdioxids wird die Reaktionsmasse nach dem Erkalten mit verdünnter Mineralsäure, organischer Säure oder einem Säuregemisch (stöchiometrischer Überschuß bis 10 Mol-%) bei Zimmertemperatur so lange umgesetzt, bis eine Suspension aus mikroskopisch kleinen Plättchen entstanden ist (Zeitdauer: sechs bis zwölf Stunden). Die Suspension wird filtriert und salzarm bis salzfrei gewaschen, getrocknet und anschließend auf das gewünschte Kornfraktionsspektrum vermahlen.

In einer Variante zum neuen Verfahren werden Natriumcarbonat und Quarzpulver in dem Molverhältnis 1:2 bis 1:3, vorzugsweise 1:2, sowie zusätzlich 0 bis 30 Gew.-%, vorzugsweise 3 bis 10 Gew.-%, Mineralisator (bezogen auf die Menge an Natriumcarbonat und Quarz), zum Beispiel Natriumchlorid, intensiv miteinander vermischt. Anschließend wird diese Mischung bei Temperaturen zwischen 900 und 1100$^{o}$C, vorzugsweise 950 und 1050$^{o}$C, eine halbe Stunde bis drei Stunden, vorzugsweise eineinhalb bis zweieinhalb Stunden, zu Glas geschmolzen. Das Glas wird innerhalb sehr kurzer Zeit abgekühlt. Das erstarrte Glas wird daraufhin vier bis neun Stunden bei Temperaturen zwischen 400 und 860$^{o}$C, vorzugsweise 650 bis 850$^{o}$C, getempert.

Zur Herstellung kristallisierter Formen des hydratisierten Siliciumdioxids wird die erhaltene Reaktionsmasse nach dem Erkalten wieder wie bei der ersten Variante mit verdünnter Mineralsäure, organischer Säure oder einem Säuregemisch (stöchiometrischer Überschuß bis 10 Mol-%) bei Zimmertemperatur so lange umgesetzt, bis eine Suspension aus kleinen Plättchen entstanden ist (Zeitdauer: sechs bis zwölf Stunden). Die Suspension wird filtriert und salzarm bis salzfrei gewaschen,getrocknet und anschließend auf das gewünschte Kornfraktionsspektrum vermahlen.

Die kristallinen Formen des hydratisierten Siliciumdioxids, die nach dem erfindungsgemäßen Verfahren hergestellt werden, sind frei von Quarz. Die Variante zum neuen Verfahren umfaßt zusätzliche Verfahrensschritte und ist dadurch etwas aufwendiger. Dadurch lassen sich Teilchengrößen des Natriumsilicats bzw. des kristallisierten hydratisierten Siliciumdioxids bis zu 1 mm gewinnen. Darüberhinaus zeigen die kristallisierten Formen des hydratisierten Siliciumdioxids nach der Variante zum neuen Verfahren bei gleichem Röntgendiagramm ein besseres Quellungsvermögen. Durch entsprechende Vermahlung können leicht äußere Oberflächen von 40 bis 100 $m^2$/g nach BET erhalten werden.

Die folgenden Beispiele erläutern die Erfindung:

Beispiel 1

27 Gewichtsteile Natriumcarbonat (Körnung 1 mm), 30 Gewichtsteile Quarzpulver mit einer Oberfläche von 0,45 $m^2$/g nach BET und 2,9 Gewichtsteile Natriumchlorid werden intensiv vermischt und anschließend in einem Porzellantiegel sechs Stunden lang bei 820°C getempert. Nach dem Röntgendiagramm handelt es sich bei dem Natriumsilicat um $\alpha$-Na$_2$Si$_2$O$_5$ (ASTM - Nr. 18-1241). Nach dem Erkalten wird das Natriumsilicat mit 284 Gewichtsteilen 2n Salzsäure umgesetzt. Nach etwa 1o Stunden ist eine Suspension entstanden, die auf einer Nutsche abfiltriert und mit 200 Gewichtsteilen destilliertem Wasser salzarm gewaschen wird. Anschließend wird der Filterkuchen in einem Trockenschrank getrocknet und auf einer Kugelmühle vermahlen.

Beispiel 2

27 Gewichtsteile Natriumcarbonat (Körnung 1 mm), 30 Gewichtsteile Quarzpulver mit einer Oberfläche von 1,3 $m^2$/g nach BET und 2,9 Gewichtsteile Natriumchlorid werden intensiv vermischt und anschließend in einem Porzellantiegel vier Stunden lang bei 820°C getempert. Nach dem Röntgendiagramm handelt es sich bei dem Natriumsilicat um $\alpha$-Na$_2$Si$_2$O$_5$ (ASTM - Nr. 18-1241). Nach dem Erkalten wird das Natriumsilicat mit 284 Gewichtsteilen 2n Salzsäure umgesetzt. Nach etwa 8 Stunden ist eine Suspension entstanden, die auf einer Nutsche abfiltriert und mit 200 Gewichtsteilen destilliertem Wasser salzarm gewaschen wird. Anschließend wird der Filterkuchen in einem Trockenschrank getrocknet und auf einer Kugelmühle vermahlen.

Beispiel 3

13,5 Gewichtsteile Natriumcarbonat (Körnung 1 mm), 20,4 Gewichtsteile 50%-iger wäßriger Natriumhydroxidlösung, 30 Gewichtsteile Quarzpulver mit einer Oberfläche von 0,45 $m^2/g$ nach BET und 2,9 Gewichtsteile Natriumchlorid werden intensiv miteinander vermischt und daraufhin bei 100°C im Trockenschrank getrocknet. Anschließend wird die Mischung in einem Porzellantiegel zwei Stunden lang bei 1000°C geschmolzen. Innerhalb sehr kurzer Zeit wird die Glasschmelze auf einer gekühlten Metallplatte abgekühlt. Das erstarrte Glas wird daraufhin bei 780°C getempert. Nach dem Röntgendiagramm handelt es sich um $\alpha - H_2Si_2O_5$ (ASTM-Nr. 181241). Nach dem Erkalten wird das Natriumsilikat mit 284 Gew.Teilen 2n Salzsäure umgesetzt. Nach etwa 1o Stunden ist eine Suspension entstanden, die auf einer Nutsche abfiltriert und mit 2oo Gew.Teilen destilliertem Wasser salzarm gewaschen wird. Anschließend wird der Filterkuchen in einem Trockenschrank getrocknet und auf einer Kugelmühle vermahlen.

Beispiel 4

27 Gewichtsteile Natriumcarbonat (Körnung 1 mm), 30 Gewichtsteile Quarzpulver mit einer Oberfläche von 0,45 $m^2/g$ nach BET und 2,9 Gewichtsteile Natriumchlorid werden intensiv miteinander vermischt und anschließend in einem Porzellantiegel zwei Stunden lang bei 1000°C zu Glas geschmolzen. Innerhalb sehr kurzer Zeit wird die Glasschmelze auf einer gekühlten Metallplatte abgekühlt. Das erstarrte Glas wird daraufhin sechs Stunden lang bei 780°C getempert. Nach dem Röntgendiagramm handelt es sich bei dem Natriumsilicat um $\alpha - Na_2Si_2O_5$ (ASTM-Nr.18-1241). Nach dem Erkalten wird das Natriumsilicat mit 284 Gewichtsteilen 2N Salzsäure umgesetzt. Nach etwa sechs Stunden wird die dabei entstandene Suspension auf einer Nutsche abfiltriert

und mit 200 Gewichtsteilen destilliertem Wasser salzarm gewaschen. Anschließend wird der Filterkuchen in einem Trockenschrank getrocknet und auf einer Kugelmühle vermahlen.

0056094


DR. REINHARD MATOUSCHEK

8920 Schongau

---

Verfahren zur Herstellung kristalliner Natriumsilicate und kristallisierter Formen des hydratisierten Siliciumdioxids, die bei dem Verfahren
erhaltenen Produkte sowie ihre Verwendung

---

P A T E N T A N S P R Ü C H E

1. Verfahren zur Herstellung kristalliner Natriumsilicate und kristallisierter Formen des hydratisierten Siliciumdioxids durch Erhitzen von Natriumcarbonat und Quarz bei erhöhter Temperatur, dadurch g e k e n n z e i c h n e t ,
daß Natriumcarbonat der Körnung bis 2 mm und Quarzpulver mit
einer Oberfläche nach BET von 0,03 m²/g bis 20 m²/g im Molverhältnis 1:1,9 bis 1:3 intensiv miteinander vermischt werden,
diese Reaktionsmischung

a) bei Temperaturen zwischen 400 und 900°C drei bis zehn
   Stunden getempert oder

b) bei Temperaturen zwischen 900 und 1100°C eine halbe bis drei Stunden zu Glas geschmolzen wird, das flüssige Glas innerhalb kurzer Zeit abgekühlt und das erstarrte Glas darauf vier bis neun Stunden bei Temperaturen zwischen 400 und 860°C getempert wird

und das nach dem Tempern und Erkalten erhaltene Natriumsilicat isoliert und vermahlen oder zur Herstellung von kristallisierten Formen des hydratisierten Siliciumdioxids mit verdünnter Mineralsäure, organischer Säure oder einem Säuregemisch umgesetzt, der entstehende Feststoffbrei abfiltriert, salzarm gewaschen, der Filterkuchen getrocknet und anschließend vermahlen wird.

2.	Verfahren nach Anspruch 1, dadurch g e k e n n - z e i c h n e t , daß das Natriumcarbonat bis 20 Gew.-% Calciumoxid und/oder Calciumcarbonat und geringe Mengen Eisen(II,III)-oxid enthält.

3.	Verfahren nach Anspruch 1 oder 2, dadurch g e - k e n n z e i c h n e t , daß dem Gemisch aus Natriumcarbonat und Quarzpulver 0 bis 30 Gew.-%, vorzugsweise 3 bis 10 Gew.-%, Alkali- oder Erdalkalihalogenide oder deren Gemische, bezogen auf die Menge an Natriumcarbonat und Quarzpulver, beigemischt werden.

4.	Verfahren nach Anspruch 1, 2 oder 3, dadurch g e - k e n n z e i c h n e t , daß das Natriumcarbonat bis zu 90 Mol-% durch Natriumhydroxid ersetzt werden kann, wobei das Natriumhydroxid in Form einer wäßrigen Lösung den übrigen Reaktionspartnern beigemischt wird und die Mischung anschließend zur Entfernung des Wassers getrocknet wird.

5.	Kristallines Natriumsilikat und kristallisierte Formen des hydratisierten Siliciumdioxids, dadurch g e - k e n n z e i c h n e t , daß die nach dem Verfahren nach einem der Ansprüche 1 bis 4 erhalten worden sind.

6.     Verwendung der kristallinen Natriumsilikate und kristallisierten Formen des hydratisierten Siliciumdioxids gemäß Anspruch 5 als verstärkende Füllstoffe in Natur- und Synthesekautschuk, Siliconkautschuk, Kunststoffen, Papier, für kosmetische und medizinische Anwendungen, als Trägerstoffe für Öle pflanzlicher und tierischer Herkunft und Biocide sowie für die selektive Adsorption und Verdickung als Katalysator und Katalysatorträger.

| | Europäisches Patentamt | **EUROPÄISCHER RECHERCHENBERICHT** | 0056094 Nummer der Anmeldung |
| --- | --- | --- | --- |

EP 81 10 9038.0

## EINSCHLÄGIGE DOKUMENTE

| Kategorie | Kennzeichnung des Dokuments mit Angabe, soweit erforderlich, der maßgeblichen Teile | betrifft Anspruch |
| --- | --- | --- |
| D,A | ZEITSCHRIFT FÜR ANORGANISCHE UND ALLGEMEINE CHEMIE, Band 335, 1965 F. WODTCKE et al. "Über die Darstellung zweier Modifikationen der Phyllokieselsäure $H_2 Si_2 O_5$" Seiten 178 bis 188 -- | |
| A | FR - A - 1 525 160 (MINES DOMANIALES DE POTASSE D'ALSACE) * Ansprüche 1 bis 4 * -- | |
| A | US - A - 3 172 727 (O.W. BURKE, JR. et al.) * Anspruch 1, Beispiel 1 * -- | |
| D,A | US - A - 4 073 865 (E.M. FLANIGEN et al.) -- | |
| D,A | DE - B2 - 2 751 443 (UNION CARBIDE CORP.) -- | |
| A | US - A - 2 239 880 (D.B. CURLL) -- | |
| A | DE - A1 - 2 519 265 (ZINKWIT NEDERLAND B.V.) ---- | |

**KLASSIFIKATION DER ANMELDUNG (Int. Cl.)**

C 01 B  33/32
C 01 B  33/12
B 01 J  21/08
B 01 J  23/02
B 01 J  20/10
C 08 K   3/36

**RECHERCHIERTE SACHGEBIETE (Int. Cl.³)**

C 01 B  33/00

**KATEGORIE DER GENANNTEN DOKUMENTE**

X: von besonderer Bedeutung allein betrachtet
Y: von besonderer Bedeutung in Verbindung mit einer anderen Veröffentlichung derselben Kategorie
A: technologischer Hintergrund
O: nichtschriftliche Offenbarung
P: Zwischenliteratur
T: der Erfindung zugrunde liegende Theorien oder Grundsätze
E: älteres Patentdokument, das jedoch erst am oder nach dem Anmeldedatum veröffentlicht worden ist
D: in der Anmeldung angeführtes Dokument
L: aus andern Gründen angeführtes Dokument

&: Mitglied der gleichen Patentfamilie, übereinstimmendes Dokument

Der vorliegende Recherchenbericht wurde für alle Patentansprüche erstellt.

| Recherchenort | Abschlußdatum der Recherche | Prüfer |
| --- | --- | --- |
| Berlin | 07-04-1982 | ASSOGNA |

EPA form 1503.1  06.78